Europäisches Patentamt

European Patent Office

Office européen des brevets

(11) Publication number: **0 141 414**
A2

# EUROPEAN PATENT APPLICATION

(21) Application number: **84113286.3**

(22) Date of filing: **05.11.84**

(51) Int. Cl.⁴: **A 61 K 7/06**

(30) Priority: **07.11.83 JP 208788/83**

(43) Date of publication of application:
**15.05.85 Bulletin 85/20**

(84) Designated Contracting States:
**DE FR GB IT**

(71) Applicant: **KAKUDAI SHOSAN KABUSHIKI KAISHA
1-4, Kita-Machi
Kawagoe-Shi Saitama-Ken(JP)**

(72) Inventor: **Someya, Kiyoshi
1-4, Kita-Machi
Kawagoe-Shi Saitama-Ken(JP)**

(74) Representative: **Patentanwälte Dipl.-Ing. Klaus Behn
Dipl.-Phys. Robert Münzhuber
Widenmayerstrasse 6/IV
D-8000 München 22(DE)**

(54) **Hair tonic composition.**

(57) A hair tonic composition containing at least one plant hormone selected from the group consisting of p-chlorophenoxyacetic acid, sodium 5-chloro-1H-3-indazolylacetate and α-naphthylacetamide as an effective ingredient in a quantity of from 0.05 % to 3.0 % by weight.

0141414

HAIR TONIC COMPOSITION

BACKGROUND OF THE INVENTION

This invention relates generally to hair tonic compositions and more particularly to a hair tonic composition which is a hair care product prepared by adding a variety of ingredients for hair tonic preparations to solvents such as ethanol and water in addition to propylene glycol, perfume, color and the like. The hair tonic composition is intended to afford the following effects: the blood circulation at the scalp is improved to enhance the skin function, whereby the hair roots are activated to prevent hair loss; the dust and grime on the scalp and hair are removed to prevent the dandruff and itch, whereby hair sprouting and hair growth are promoted; and a refreshing feeling is imparted.

While the physiological mechanism with regard to hair loss, hair sprouting and hair growth is not apparent in many respects, the following ingredients for hair tonic preparations have been heretofore used:

1) Hormones such as follicular hormone, adrenal cortical hormone and the like;

2) Vitamins such as vitamins E, $B_2$ and $B_6$ and the like;

3) Amino acids;

4) Crude during extracts such as Japanese chirata extract and the like;

5) Antiphlogistics such as diphenhydramine hydrochloride, glycyrrhizin and the like;

6) Keratin-solubilizing agents such as lactic acid, resorcinol, salicylic acid and the like;

7) Scalp-stimulating agents such as benzyl nicotinate, tincture of copricum, nonylic vanilamide, tincture of cantharis and the like;

8) Germicides such as isopropyl methylphenol, alkyldiaminoethyl glycine hydrochloride solution, p-chloro-m-xylenol, quaternary ammonium salts and the like;

9) Refrigerants such as menthol and the like; and

10) Humectants such as glycerin, propylene glycol, sorbitol, sodium dl-pyrrolidonecarboxylate and the like.

However, because the use of a large amount of certain ingredients for half tonic preparations such as hormones, germicides and scalp-stimulating agents may cause drawbacks with regard to safety, it is strictly required that the amount of such ingredients incorporated into the hair tonic composition be accurately adjusted. Further, certain ingredients for hair tonic preparations are not entirely satisfactory with respect to prevention of hair loss as well as hair sprouting – and hair growth – promoting effects.

I have made extensive studies in order to overcome the drawbacks accompanying such prior art hair tonic compositions.

### SUMMARY OF THE INVENTION

It is an object of the present invention to provide a hair tonic composition which is safe and which has various advantages such as the promotion of hair sprouting and hair growth.

A hair tonic composition according to the present invention comprises at least one plant hormone, or phytohormone, selected from the group consisting of p-chlorophenoxyacetic acid, sodium 5-chloro-1H-3-indazolylacetate and α-naphthylacetamide as an effective ingredient.

First, p-chlorophenoxyacetic acid has been heretofore used as a plant growth regulator in order to increase the bearing of tomatoes, eggplants and the like at low temperatures, to fatten the fruit and to shorten the maturation period. Particularly, p-chlorophenoxyacetic acid has been known as a compound serving to secure the yield of fruits in the case of forced

3

0141414

culture or semi-forced culture and to improve the quality thereof. However, it was not known at all that hair sprouting and hair growth-promoting effects could be obtained by using p-chlorophenoxyacetic acid in a hair tonic composition.

Further, α-naphthylacetamide has been heretofore used as a plant growth regulator in order to promote root sprouting or activation of cuttings (or saplings grown from the cutting) of petal plants such as chrysanthemums, roses and geraniums; garden trees such as spindle trees, daphnes, Japanese laurels, azaleas, rhododendrons, camellias, and pines; trees such as cryptomeria and sun trees. However, it was not known at all that hair sprouting - and hair growth - promoting effects could be obtained by using α-naphthylacetamide in a hair tonic composition.

Furthermore, sodium 5-chloro-1H-3-indazolylacetate has been heretofore used as a plant growth regulator in order to promote root sprouting or activation of cuttings of flowering trees such as Japanese hollys, Enkianthus sweet osmanthus, Juniperus Chinensis camellias and Himalayan cedars. However, it was not known at all that the prevention of hair loss, dandruff and itch as well as hair sprouting - and hair growth - promoting effects could be obtained by using sodium 5-chloro-1H-3-indazolylacetate in a hair tonic composition.

### DETAILED DESCRIPTION OF THE INVENTION

When p-chlorophenoxyacetic acid, sodium 5-chloro-1H-3-indazolylacetate or α-naphthylacetamide is used alone in the present invention, each compound is present in the hair tonic composition at a level of from 0.001 % to 5 % by weight, preferably from 0.05 % to 3.0 % by weight. Further, when a mixture of the compounds described above is used, the compounds are present in the hair tonic composition in a total amount of from 0.001 % to 5 % by weight, preferably from 0.05 % to 3.0 % by weight.

In addition to p-chlorophenoxyacetic acid, sodium 5-chloro-1H-3-indazolylacetate and α-naphthylacetamide, the hair tonic composition according to the present invention can contain various ingredients conventionally used in hair tonic compositions. Examples of such optional ingredients are solvents such as purified water and ethanol; refrigerants such as menthol; humectants such as propylene glycol, glycerin and sorbitol; keratin-solubilizing agents such as lactic acid, resorcinol and salicyclic acid; ethers such as polyoxyethylene oleyl ether and polyoxyethylene lauryl ether; vegetable oils such as olive oil and castor oil; higher alcohols such as cetyl alcohol and oleyl alcohol; hydrocarbons such as liquid paraffin and octene; vitamins such as vitamin E; salts such as sodium chloride; perfumes; coloring agents; and preservatives.

If necessary, other ingredients for hair tonic preparations such as hormones such as follicular hormone; antiphlogistics such as diphenhydramine hydrochloride; germicides such as alkyldiaminoethyl glycine hydrochloride; and scalp-stimulating agents such as benzyl nicotinate can be incorporated in the hair tonic composition of the present invention.

Further, hair sprouting - and hair growth - promoting effects can be enhanced by using at least one plant hormone selected from the group consisting of p-chlorophenoxyacetic acid, sodium 5-chloro-1H-3-indazolylacetate and α-naphthylacetamide in combination with proteolytic enzymes such as papain, ficin, bromelin and pepsin. Furthermore, it is thought that the reduction of the activity of p-chlorophenoxyacetic acid, sodium 5-chloro-1H-3-indazolylacetate, α-naphthylacetamide or mixtures thereof can be prevented by incorporating a small amount of sodium chloride in the hair tonic composition of the present invention.

The hair tonic composition according to the present invention can be prepared in accordance with the conventional process for preparing an ordinary hair tonic composition except that at least one plant hormone selected from the group consisting of p-chlorophenoxyacetic acid, sodium 5-chloro-1H-3-indazolylacetate and α-naphthylacetamide is incorporated therein.

The following examples illustrate the present invention but are not intended to limit the scope thereof. Throughout these examples, quantities expressed in "parts" are by weight.

### EXAMPLE 1

A hair tonic composition containing the following ingredients was prepared.

| | |
|---|---|
| Ethanol | 55 parts |
| Propylene glycol | 13 parts |
| Sodium chloride | 2 parts |
| p-Chlorophenoxyacetic acid | 1.0 part |
| Color, perfume | q.s. |
| Purified water | 25 parts |

When this hair tonic composition was applied daily onto the scalps of Japanese subjects with thin hair and the scalps were well massaged in the morning and evening over 3 months, dandruff and itch were prevented, and it was observed that downy hair began to sprout on the back and front of the head after about 3 weeks. This downy hair gradually became tinged with black.

### EXAMPLE 2

A hair tonic composition containing the following ingredients was prepared.

| | |
|---|---|
| Ethanol | 70 parts |
| Vitamin E | 0.1 part |
| Propylene glycol | 3 parts |
| Menthol | 0.1 part |
| Diphenhydramine hydrochloride | 0.01 part |
| Lactic acid | 0.1 part |
| p-Chlorophenoxyacetic acid | 0.5 part |

| Sodium chloride | 2 parts |
| Color, perfume | q.s. |
| Purified water | 30 parts |

When this hair tonic composition was applied daily onto the scalps of Japanese subjects with thin hair and the scalps were well massaged in the morning and evening over 3 months, dandruff and itch were prevented, and it was observed that new downy hair began to sprout in the vicinity of the back of the head after about 3 weeks.

EXAMPLE 3

A hair tonic composition containing the following ingredients was prepared.

| Ethanol | 70 parts |
| Vitamin E | 0.1 part |
| Propylene glycol | 3 parts |
| Menthol | 0.1 part |
| Diphenhydramine hydrochloride | 0.01 part |
| p-Chlorophenoxyacetic acid | 1.0 part |
| Papain | 0.5 part |
| Sodium chloride | 2 parts |
| Color, perfume | q.s. |
| Purified water | 25 parts |

When this hair tonic composition was applied daily onto the scalps of Japanese subjects with thin hair and the scalps were well massaged in the morning and evening over 3 months, dandruff and itch were prevented, and it was observed that new downy hair began to sprout in the vicinity of the back of the head after about 4 weeks.

EXAMPLE 4

A hair tonic composition containing the following ingredients was prepared.

| Ethanol | 55 parts |
| Propylene glycol | 13 parts |
| Sodium chloride | 2 parts |
| Sodium 5-chloro-1H-3-indazolyl-acetate | 1.0 part |

0141414

| | |
|---|---|
| Color, perfume | q.s. |
| Purified water | 25 parts |

When this hair tonic composition was applied daily onto the scalps of Japanese subjects with thin hair and the scalps were well massaged in the morning and evening over 3 months, dandruff and itch were prevented, and it was observed that downy hair began to sprout on the back and front of the head after about 3 weeks. This downy hair gradually became tinged with black.

## EXAMPLE 5

A hair tonic composition containing the following ingredients was prepared.

| | |
|---|---|
| Ethanol | 70 parts |
| Vitamin E | 0.1 part |
| Propylene glycol | 3 parts |
| Menthol | 0.1 part |
| Diphenhydramine hydrochloride | 0.01 part |
| Lactic acid | 0.1 part |
| Sodium 5-chloro-1H-3-indazolylacetate | 0.5 part |
| Sodium chloride | 2 parts |
| Color, perfume | q.s. |
| Purified water | 30 parts |

When this hair tonic composition was applied daily onto the scalps of Japanese subjects with thin hair and the scalps were well massaged in the morning and evening over 3 months, dandruff and itch were prevented, and it was observed that new downy hair began to sprout in the vicinity of the back of the head after about 3 weeks.

## EXAMPLE 6

A hair tonic composition containing the following ingredients was prepared.

| | |
|---|---|
| Ethanol | 70 parts |
| Vitamin E | 0.1 part |
| Propylene glycol | 3 parts |
| Menthol | 0.1 part |

| Diphenhydramine hydrochloride | 0.01 | part |
| Sodium 5-chloro-1H-3-indazolylacetate | 1.0 | part |
| Papain | 0.5 | part |
| Sodium chloride | 2 | parts |
| Color, perfume | q.s. | |
| Purified water | 25 | parts |

When this hair tonic composition was applied daily onto the scalps of Japanese subjects with thin hair and the scalps were well massaged in the morning and evening over 3 months, dandruff and itch were prevented, and it was observed that new downy hair began to sprout in the vicinity of the back of the head after about 4 weeks.

EXAMPLE 7

A hair tonic composition containing the following ingredients was prepared.

| Ethanol | 55 | parts |
| Propylene glycol | 13 | parts |
| Sodium chloride | 2 | parts |
| α-Naphthylacetamide | 1.0 | part |
| Color, perfume | q.s. | |
| Purified water | 25 | parts |

When this hair tonic composition was applied daily onto the scalps of Japanese subjects with thin hair and the scalps were well massaged in the morning and evening over 3 months, dandruff and itch were prevented, and it was observed that downy hair began to sprout on the back and front of the head after about 3 weeks. This downy hair gradually became tinged with black.

EXAMPLE 8

A hair tonic composition containing the following ingredients was prepared.

| Ethanol | 70 | parts |
| Vitamin E | 0.1 | part |
| Propylene glycol | 3 | parts |
| Menthol | 0.1 | part |
| Diphenhydramine hydrochloride | 0.01 | part |

| Lactic acid | 0.1 part |
| α-Naphthylacetamide | 0.5 part |
| Sodium chloride | 2 parts |
| Color, perfume | q.s. |
| Purified water | 30 parts |

When this hair tonic composition was applied daily onto the scalps of Japanese subjects with thin hair and the scalps were well massaged in the morning and evening over 3 months, dandruff and itch were prevented, and it was observed that new downy hair began to sprout in the vicinity of the back of the head after about 3 weeks.

### EXAMPLE 9

A hair tonic composition containing the following ingredients was prepared.

| Ethanol | 70 parts |
| Vitamin E | 0.1 part |
| Propylene glycol | 3 parts |
| Menthol | 0.1 part |
| Diphenhydramine hydrochloride | 0.01 part |
| α-Naphthylacetamide | 1.0 part |
| Papain | 0.5 parts |
| Sodium chloride | 2 parts |
| Color, perfume | q.s. |
| Purified water | 25 parts |

When this hair tonic composition was applied daily onto the scalps of Japanese subjects with thin hair and the scalps were well massaged in the morning and evening over 3 months, dandruff and itch were prevented and it was observed that new downy hair began to sprout in the vicinity of the back of the head after about 3 weeks.

## WHAT IS CLAIMED IS:

1.    A hair tonic composition comprising at least one plant hormone, or phytohormone, selected from the group consisting of p-chlorophenoxyacetic acid, sodium 5-chloro-1H-3-indazolylacetate and α-naphthylacetamide as an effective ingredient.

2.    The hair tonic composition according to claim 1 wherein said plant hormone is p-chlorophenoxyacetic acid.

3.    The hair tonic composition according to claim 1 wherein said plant hormone is sodium 5-chloro-1H-3-indazolylacetate.

4.    The hair tonic composition according to claim 1 wherein said plant hormone is α-naphthylacetamide.

5.    The hair tonic composition according to claim 1 wherein said plant hormone is present in the composition in a quantity of from 0.001 % to 5 % by weight.

6.    The hair tonic composition according to claim 5 wherein said plant hormone is present in the composition in a quantity of from 0.05 % to 3.0 % by weight.

7.    The hair tonic composition according to claim 1 which additionally contains a proteolytic enzyme selected from the group consisting of papain, ficin, bromelin and pepsin.

8.    The hair tonic composition according to claim 1 which additionally contains sodium chloride.